# FASCICULE DE BREVET EUROPEEN

(11) **EP 0 323 331 B1**
(45) Date de publication et mention de la délivrance du brevet: **20.03.1996**
(21) Numéro de dépôt: 88403297.0
(22) Date de dépôt: 22.12.1988
(51) Int. Cl.: G01N 33/569

(54) **Réactif pour la détection de Staphylococcus aureus par agglutination**
Reagens zum Nachweis von Staphylococcus aureus durch Agglutination
Reagent for the detection of Staphylococcus aureus by agglutination

(30) Priorité: 24.12.1987 FR 8718166
(43) Date de publication de la demande: 05.07.1989
(73) Titulaire: INSTITUT PASTEUR, F-75724 Paris Cédex 15 (FR)
(72) Inventeur: Fournier, Jean-Michel, 75018 Paris (FR); Boutonnier, Alain, 75013 Paris (FR)
(74) Mandataire: Le Guen, Gérard

(56) Documents cités:
- DD-A- 239 006
- FR-A- 2 420 572
- FR-A- 2 537 725
- INFECTION AND IMMUNITY, vol. 49, no. 1, juillet 1985, US; M.J.NELLES et al., pp. 14-18*
- CHEMICAL ABSTRACTS, vol. 104, no. 11, mars 1986, Columbus, OH (US); p. 339, no.
- 84910y*
- JOURNAL OF CLINICAL MICROBIOLOGY, 1987; pp. 1932-1933*
- JOURNAL OF CLINICAL MICROBIOLOGY, 1989; pp. 1372-1374*
- JOURNAL OF CLINICAL MICROBIOLOGY, 1986; pp. 490-491*
- U. FORSUM et al., Infect. Immun., October 1972, pp. 583-586
- J.M. FOURNIER et al., J. Clin. Microbiol., Vol. 25/10, Octobre 1987, pp. 1932-1933
- J.M. FOURNIER et al., J. Clin. Microbiol., Vol. 27/6, Juin 1989, pp. 1372-1374
- J. RUANE et al., J. Clin. Microbiol., Vol. 24/3, Septembre 1986, pp. 490-491

## Description

La présente invention concerne un réactif pour la détection par agglutination, de Staphylococcus aureus.

On connaît déjà divers réactifs pour la détection de Staphylococcus aureus. Ces réactifs sont basés sur la recherche soit de la protéine A du Staphylocoque, soit du facteur d'affinité pour le fibrinogène, soit des deux simultanément.

La protéine A est un antigène de nature protéique, constituant externe de la paroi de la majorité des souches de Staphylococcus aureus d'origine humaine (85 à 95%). La protéine A fixe, par un procédé non immunologique, le fragment Fc des immunoglobulines, laissant libre la partie Fab.

Si l'on met en présence des souches de Staphylococcus aureus possédant la protéine A et des hématies de mouton, ou des particules de latex, sensibilisées, par exemple avec du sérum de lapin anti-hématies de mouton, on observe en quelques minutes, une agglutination visible à l'oeil nu.

Le facteur d'affinité pour le fibrinogène, qui est fixé à la surface de Staphylococcus aureus réagit directement avec le fibrinogène. Ce facteur d'affinité pour le fibrinogène peut être recherché, en quelques secondes, en mettant en contact la souche à étudier et des hématies de mouton (hémagglutination passive) ou des particules de latex, les unes ou les autres recouvertes de fibrinogène.

Différentes études publiées montrent qu'un certain nombre de souches de Staphylococcus aureus ne sont pas identifiées par ces réactifs. Leur pourcentage varie de 1 à 5% lorsque ces études sont réalisées sur l'ensemble des Staphylocoques dorés isolés. Mais ce pourcentage d'échec est plus important lorsque seules les souches résistantes à l'oxacilline (ou la méticilline) sont prises en considération et ce pourcentage atteint 25% dans une étude récente réalisée avec 73 souches de Staphylococcus aureus résistantes à l'oxacilline (voir P. J. Ruane et al, J. Clin. Microbiol. 24, 490, 1986).

M.J. NELLES ET AL., INFECTION AND IMMUNITY, vol. 49, n° 1, juillet 1985, pages 14-18 rapporte les résultats d'études de réactivité d'anticorps monoclonaux spécifiques d'un type donné de polysaccharide capsulaire avec des isolats cliniques de Staphylococcus aureus et des polysaccharides de surface masquent fonctionnellement les antigènes de la paroi cellulaire.

D'autre part, Fournier et al (Journal of Clinical Microbiology, Oct. 87, p. 1932-1933) ont montré que parmi les souches de Staphylococcus aureus résistantes à l'oxacilline existe une prédominance de polyosides capsulaires de type 5.

Les auteurs rapportent également que les résultats des tests de typage ELISA, montrent que parmi les souches résistantes à l'oxacilline, environ 5% ne peuvent être typées à l'aide d'anticorps monoclonaux reconnaissant soit le polyoside capsulaire de type 5 soit le polyoside capsulaire de type 8.

Infection Immunity, 1972, pp. 583-586 décrit que la formation de colonies compactes de Staphylococcus aureus sur gélose-sérum est principalement le résultat d'une réaction entre la protéine A et le fragment Fc des IgG.

Cependant, à ce jour aucune étude n'a abouti à la mise au point d'un réactif capable de détecter toutes les souches de Staphylococcus aureus.

Dans une étude réalisée, sous la direction de l'un des inventeurs, sur 183 souches de Staphylococcus aureus isolées de malades dans 5 hôpitaux de Paris et de la région parisienne, on a trouvé que 7 souches (4%) ne sont agglutinées par aucun des trois réactifs utilisés (Staphyslide^{R}, Staphaurex^{R} et Pastorex^{R} Staph). Si on ne considère que les 50 souches résistantes à l'oxacilline, on constate que 6 souches (12%) sont faussement négatives avec ces réactifs du commerce. Ces résultats sont donc en accord avec ceux décrits dans la littérature.

Deux hypothèses peuvent être envisagées pour expliquer le fait que certaines souches de Staphylococcus aureus ne sont pas agglutinées par les réactifs du commerce :
1) Ces souches produisent la protéine A en quantité trop faible pour qu'il y ait, entre cette protéine et les particules de latex, une réaction suffisante pour provoquer une agglutination bactérienne.
2) La protéine A est produite en quantité normale, mais cet antigène, ainsi que le facteur d'affinité pour le fibrinogène, sont recouverts par un ou plusieurs autres antigènes et sont donc inaccessibles aux particules de latex.

Dans une étude réalisée sous la direction de l'un des inventeurs, il a été montré que les souches non agglutinées par les particules de latex produisent autant de protéine A que les autres souches. Ce résultat permet donc d'écarter la première hypothèse. Il a été ensuite montré, en dosant le polyoside capsulaire au moyen d'une réaction immunoenzymatique utilisant des anticorps monoclonaux, que toutes les souches non agglutinées par les particules de latex sensibilisées par le fibrinogène et vis-à-vis de la protéine A possèdent du polyoside capsulaire. Enfin, les antigènes exposés à la surface du staphylocoque, et pouvant en conséquence réagir avec les particules de latex, ont été étudiés en immunofluorescence. Cette étude a porté d'une part sur la protéine A (en utilisant du fragment Fc d'immunoglobuline humaine et des fragments F(ab′)₂ pepsinés d'immunoglobulines de mouton anti-Fc humain marqués à la fluorescéine) et, d'autre part, sur le polyoside capsulaire (en utilisant des anticorps monoclonaux murins d'isotype M spécifiques du polyoside capsulaire et des fragments F(ab′)₂ d'immunoglobulines de chèvre antiimmunoglobulines M de souris marqués à la rhodamine). Cette étude a clairement montré que la protéine A n'est pas exposée, ou alors en très faible quantité, à la surface des bactéries qui ne sont pas agglutinées par le latex, et que la surface de ces bactéries est par contre totalement recouverte par le polyoside capsulaire. A titre de contrôle, il a été vérifié que les souches qui sont agglutinées par les particules de latex exposent bien à leur surface la protéine A.

Cette étude montre donc que le polyoside capsulaire synthétisé par certaines souches de Staphylococcus aureus recouvre toute la bactérie, masque les antigènes pouvant être reconnus par les réactifs du commerce, et empêche ainsi l'identification de ces souches comme appartenant à l'espèce Staphylococcus aureus du fait de leur agglutination par les réactifs du commerce.

Le résultat de cette étude et en particulier le fait que les souches de Staphylococcus aureus qui ne présentent pas de protéine A à la surface sont des souches capsulées dont les polyosides capsulaires masquent la protéine A a permis de concevoir un réactif pour la détection des souches de Staphylococcus aureus qui présente une fiabilité plus grande que les réactifs connus.

La présente invention a ainsi pour objet un réactif pour la détection par agglutination de Staphylococcus aureus, du type comprenant des particules en suspension sur lesquelles sont fixés du fibrinogène et des anticorps reconnaissant par affinité la protéine A du staphylocoque, caractérisé en ce qu'il comprend des particules en suspension sur lesquelles sont fixés au moins un anticorps reconnaissant de façon spécifique un polyoside capsulaire de Staphylococcus aureus de type 5.

La présente invention a également pour objet une procédé de détection par agglutination de Staphylococcus aureus dans un échantillon, qui consiste à mélanger l'échantillon avec un réactif selon l'invention et à observer s'il se produit une agglutination.

A ce jour 11 types de polyosides capsulaires ont été identifiés par des méthodes essentiellement immunologiques. Voir à ce sujet : W. W. Karakawa et al. Capsular polysaccharides of Staphylococcus aureus p. 285-293. In J.B. Robbins, J.C. Hill, and J.C. Sadoff (ed.) Seminars in infectious disease. vol. 4. Bacterial vaccines. Thieme Stratton. Inc. New York; W. W. Karakawa et al. J. Clin. Microbiol. 22 : 445-447, 1985; Sompolinsky et al, J. Clin. Microbiol. 22 : 828-834, 1985.

La purification et la caractéristique biochimique et immunologique du polyoside capsulaire de type 8 ont été réalisées en 1984 (J.M. Fournier et al, Infect. Immun. 45 : 87-93) et celles du type 5 en 1987 (J. M. Fournier et al, Ann. Inst. Pasteur/Microbiol. 138 : 561-567).

Des anticorps monoclonaux spécifiques des polyosides capsulaires 5 et 8 ont été décrits (H.K. Hochkeppel et al. J. Clin. Microbiol. 25 : 526-530, 1987, et M. J. Nelles et al. Infect. Immun. 49 : 14-18, 1985).

Par ailleurs des études épidémiologiques réalisées sur un grand nombre de souches de Staphylococcus aureus isolées de malades, ont montré que 70 à 80% de ces souches possédaient l'un ou l'autre des polyosides capsulaires 5 et 8 (par exemple R.D. Arbeit et al. Diagn. Microbiol. Infect. Dis. 2 : 85-91, 1984).

Aussi dans la présente invention les anticorps reconnaissant un polyoside capsulaire de Staphylococcus aureus sont constitués avantageusement au moins par des anticorps reconnaissant un polyoside capsulaire de type 5 et de préférence à la fois par des anticorps reconnaissant un polyoside capsulaire de type 5 et des anticorps reconnaissant un polyoside capsulaire du type 8.

Mais il va de soi que le réactif de diagnostic le plus fiable comprend un ensemble d'anticorps reconnaissant les différents types de polyosides capsulaires.

Dans le réactif selon l'invention, les différents anticorps et le fibrinogène peuvent être fixés sur une suspension unique de particules ou bien être fixés sur différentes suspensions de particules (à raison d'un ou plusieurs types de constituant par suspension de particules) qui sont ensuite mélangées pour constituer le réactif.

Les particules en suspension utilisées dans le réactif selon l'invention sont notamment des particules de latex telles que des billes de polystyrène ou des particules similaires, ayant de préférence une taille inférieure à 2 micromètres. A titre d'exemple on peut citer les particules ESTAPOR commercialisées par la Société Rhône-Poulenc telles que
- des particules de polystyrène K 109, ayant un diamètre de 0,8 micromètre,
- et des particules de polystyrène ayant des groupes carboxyliques, PSI 480, ayant un diamètre de 0,8 micromètre.

On peut également utiliser des gels magnétiques tels que les gels de polyacrylamide et/ou d'agarose contenant des particules magnétiques qui sont décrits dans FR-A-2 334 106. On peut en outre utiliser des gels tels que Ultrogel® et Magnogel® de la Société IBF.

Les anticorps utilisés dans la présente invention peuvent être des anticorps animaux ou humains, polyclonaux ou monoclonaux.

Les anticorps reconnus par affinité par la protéine A du staphylocoque sont notamment des anticorps de classe IgG. Ils peuvent être remplacés par des fragments Fc de ces immunoglobulines.

Dans le cas d'anticorps polyclonaux, on peut utiliser pour la préparation du réactif un plasma humain ou animal, normal ou immunisé, contenant ces anticorps ou des anticorps purifiés selon les techniques classiques.

Dans le cas d'anticorps monoclonaux, on peut utiliser un surnageant de culture d'hybridome ou de liquide d'ascite préparé sur souris, ou des anticorps à l'état purifié.

Pour fixer le fibrinogène, on peut utiliser un plasma humain ou animal, normal ou hyperimmunisé, ou du fibrinogène purifié selon les techniques classiques.

La fixation de ces molécules sur les particules en suspension, généralement un latex, peut être réalisée de diverses façons :
- la fixation peut être spontanée au cours d'une incubation des particules de latex dans une solution contenant ces molécules, par exemple une incubation de 30 minutes à 56°C est souvent suffisante.
- cette fixation peut aussi être réalisée en créant une liaison covalente entre les anticorps et les groupements carboxyliques présents sur certaines particules de latex (ESTAPOR PSI 480). Il est possible d'utiliser par exemple un carbodiimide pour créer la liaison covalente.

La concentration des molécules à fixer sur les particules de latex, qui doit être déterminée pour chaque molécule selon les méthodes connues, est habituellement inférieures à 200 microgrammes par mg de latex. Dans le cas de l'utilisation de molécules en tant que constituants d'un plasma, une dilution de ce plasma au 1/1000 peut être utilisée.

Les exemples suivants illustrent la présente invention.

### Exemple 1

a) Préparation de particules de latex sensibilisées avec du fibrinogène et des anticorps contenus dans du plasma humain, y compris des IgG.

Une suspension de latex (ESTAPOR, K 109) est amenée à la concentration de 2% dans un tampon glycine (0, 27 M) - chlorure de sodium (0, 15 M) - azoture de sodium (0,04 g/litre) dit CGA, pH 8. Un volume de cette suspension est mélangé avec un volume égal de plasma humain dilué à 1/1000 dans le même tampon, puis mis à incuber 30 minutes à 56°C sous agitation. Après lavage, les particules de latex sensibilisées sont remises en suspension dans un tampon CGA contenant 0,05 % de plasma humain.

b) Préparation de particules de latex sensibilisées avec des anticorps monoclonaux purifiés reconnaissant le polyoside capsulaire de type 8.

Une suspension de latex (ESTAPOR, réf. K 109) est amenée à la concentration de 2% dans un tampon glycine (0, 17 M) - chlorure de sodium (0, 15 M) - azoture de sodium (0,04 g/litre) dit CGA, pH 9,2. Un volume de cette suspension est mélangé avec un volume égal d'anticorps purifiés, solubilisés dans le même tampon. La concentration finale d'anticorps est de 100 microgrammes d'anticorps par mg de latex. Après 30 minutes d'incubation à 37°C, les particules de latex sensibilisées sont lavées puis remises en suspension dans le tampon CGA contenant 0, 1% de sérum albumine bovine

c) Préparation du réactif.

On mélange la suspension obtenue en a) et la suspension obtenue en b) dans des proportions égales pour constituer le réactif.

### Exemple 2

On opère comme à l'exemple 1 mais en utilisant dans le stade b) un mélange d'anticorps monoclonaux reconnaissant de façon spécifique les polyosides de types 8 et 5.

A cet effet une suspension de latex Estapor (réf. K109) à la concentration de 4% dans un tampon glycine 0,15 M/litre, NaCl 0,15 M/litre, pH 8,2 (GN) est mélangé à un volume de tampon GN contenant un mélange d'anticorps monoclonal anti type 5 et anti type 8 en proportion égale à une concentration finale de 1 mg/ml. Ce mélange est incubé sous agitation 1/2 heure à 37°C.

Un volume de ce latex sensibilisé est mélangé à un tampon GN contenant 3 mg/ml d'albumine bovine et 0,04 g/l d'azoture de sodium. Ce mélange est incubé 1/2 heure à 37°C. 1 volume de ce latex anti type 5 et 8 est mélangé à 1 volume de particules de latex sensibilisées avec du fibrinogène et des anticorps du plasma humain.

### Exemple 3

On opère comme à l'exemple 1 mais en utilisant dans le stade b) un mélange de 11 anticorps reconnaissant de façon spécifique les 11 polyosides capsulaires connus de Staphylococcus aureus.

### Exemple 4

Dans une étude réalisée sous la direction de l'un des inventeurs, le pouvoir agglutinant d'un réactif, préparé selon le procédé décrit à l'exemple 2, a été comparé avec celui d'un réactif commercial (Pastorex® Staph). Cette comparaison a été réalisée avec 183 souches de Staphylococcus aureus isolées de malades. Alors que le réactif commercial n'agglutine que 176 souches sur 183, le réactif préparé selon l'exemple 2 agglutine les 183 souches. En outre, la rapidité et l'intensité de l'agglutination des souches qui étaient déjà agglutinées par le réactif commercial sont très nettement améliorées par l'utilisation du réactif préparé selon l'exemple 2.

Les réactifs selon l'invention peuvent être utilisés pour une identification rapide de l'espèce Staphylococcus aureus par agglutination :
- avant isolement de la bactérie :
   . dans un liquide biologique (sécrétion, suppuration, etc ...),
   . dans un milieu de culture (hémoculture, etc ...),
- après isolement de la bactérie.

Les réactifs selon l'invention permettent de reconnaître toutes les souches de Staphylococcus aureus qui ne sont pas reconnues par les réactifs agglutinants existant actuellement sur le marché, et parmi lesquelles prédominent les souches résistantes à l'oxacilline. Ils présentent donc un intérêt majeur compte tenu des problèmes que posent ces souches qui sont aussi le plus souvent multirésistantes.

## Revendications

1. Réactif pour la détection par agglutination de Staphylococcus aureus, du type comprenant des particules en suspension sur lesquelles sont fixés des anticorps reconnaissant par affinité la protéine A du staphylocoque et du fibrinogène, caractérisé en ce qu'il comprend des particules en suspension sur lesquelles sont fixés des anticorps reconnaissant de façon spécifique un polyoside capsulaire de Staphylococcus aureus de type 5.

2. Réactif selon la revendication 1, caractérisé en ce qu'il comprend en outre des anticorps reconnaissant un polyoside capsulaire de Staphylococcus aureus de type 8.

3. Réactif selon la revendication 1, caractérisé en ce qu'il comprend en outre un ensemble d'anticorps reconnaissant les différents autres types de polyosides capsulaires de Staphylococcus aureus.

4. Réactif selon l'une quelconque des revendications 1 à 3, caractérisé en ce que les particules en suspension sont des particules de latex.

5. Réactif selon l'une quelconque des revendications 1 à 4, caractérisé en ce que les particules ont une taille inférieure à 2 micromètres.

6. Réactif selon l'une quelconque des revendications 1 à 3, caractérisé en ce que les particules en suspension sont des gels magnétiques.

7. Procédé de détection, par agglutination, de Staphylococcus aureus dans un échantillon, caractérisé en ce que l'on mélange l'échantillon à étudier avec un réactif selon l'une quelconque des revendications précédentes et l'on observe s'il se produit une agglutination.

8. Procédé selon la revendication 7, dans lequel l'échantillon est un échantillon d'hémoculture.

## Patentansprüche

1. Reagens zum Nachweis von *Staphylococcus aureus* durch Agglutination, eines Typs umfassend suspendierte Teilchen, auf denen Antikörper, die das Protein A des Staphylococcus durch Affinität erkennen, und Fibrinogen gebunden sind, dadurch gekennzeichnet, daß es suspendierte Teilchen umfaßt, auf denen Antikörper gebunden sind, die ein Kapsel-Polyosid von *Staphylococcus aureus* des Typs 5 spezifisch erkennen.

2. Reagens nach Anspruch 1, dadurch gekennzeichnet, daß es außerdem Antikörper umfaßt, die ein Kapsel-Polyosid von *Staphylococcus aureus* des Typs 8 erkennen.

3. Reagens nach Anspruch 1, dadurch gekennzeichnet, daß es außerdem eine Gesamtheit von Antikörpern umfaßt, die die verschiedenen anderen Typen von Kapsel-Polyosiden von *Staphylococcus aureus* erkennen.

4. Reagens nach einem der Ansprüche 1 bis 3, dadurch gekennzeichnet, daß die suspendierten Teilchen Latexteilchen sind.

5. Reagens nach einem der Ansprüche 1 bis 4, dadurch gekennzeichnet, daß die Teilchen eine Größe von weniger als 2 µm aufweisen.

6. Reagens nach einem der Ansprüche 1 bis 3, dadurch gekennzeichnet, daß die suspendierten Teilchen magnetische Gele sind.

7. Verfahren zum Nachweis von *Staphylococcus aureus* in einer Probe durch Agglutination, dadurch gekennzeichnet, daß die zu untersuchende Probe mit einem Reagens nach einem der vorstehenden Ansprüche gemischt und beobachtet wird, ob sich eine Agglutination bildet.

8. Verfahren nach Anspruch 7, wobei die Probe eine Blutkulturprobe ist.

## Claims

1. Reagent for the detection by agglutination of Staphylococcus aureus, of the type comprising particles in suspension on which are fixed antibodies recognising by affinity the protein A of the staphylococcus and of the fibrinogen, characterised in that it comprises particles in suspension on which are fixed antibodies recognising in a specific manner a capsular polysaccharide of Staphylococcus aureus of type 5.

2. Reagent according to Claim 1, characterised in that in addition it comprises antibodies recognising a capsular polysaccharide of Staphylococcus aureus of type 8.

3. Reagent according to Claim 1, characterised in that in addition it comprises a set of antibodies recognising the various other types of capsular polysaccharides of Staphylococcus aureus.

4. Reagent according to any one of Claims 1 to 3, characterised in that the particles in suspension are latex particles.

5. Reagent according to any one of Claims 1 to 4, characterised in that the particles have a size less than 2 micrometers.

6. Reagent according to any one of Claims 1 to 3, characterised in that the particles in suspension are magnetic gels.

7. Method for detection, by agglutination, of Staphylococcus aureus in a sample, characterised in that one mixes the sample to be studied with a reagent according to any one of the preceding Claims and one observes if an agglutination occurs.

8. Method according to Claim 7, in which the sample is a hemoculture sample.
